(19) Europäisches Patentamt
European Patent Office
Office européen des brevets

(11) Veröffentlichungsnummer: **0 046 192**
**B1**

(12) # EUROPÄISCHE PATENTSCHRIFT

(45) Veröffentlichungstag der Patentschrift: **03.10.84**

(51) Int. Cl.³: **C 07 D 231/12,** C 07 D 249/04, C 07 D 249/06

(21) Anmeldenummer: **81105449.3**

(22) Anmeldetag: **13.07.81**

(54) **Verfahren zur gleichzeitigen Herstellung von Pyrazol und Triazolen.**

(30) Priorität: **20.08.80 DE 3031347**

(43) Veröffentlichungstag der Anmeldung: **24.02.82 Patentblatt 82/8**

(45) Bekanntmachung des Hinweises auf die Patenterteilung: **03.10.84 Patentblatt 84/40**

(84) Benannte Vertragsstaaten: **BE CH DE FR GB LI**

(56) Entgegenhaltungen:
**DE - A - 1 695 116**
**DE - C - 557 338**
**DE - C - 557 814**

(73) Patentinhaber: **BASF Aktiengesellschaft, Carl-Bosch-Strasse 38, D-6700 Ludwigshafen (DE)**

(72) Erfinder: **Rieber, Norbert, Dr., Liebfrauenstrasse 1C, D-6800 Mannheim (DE)**
Erfinder: **Platz, Rolf, Dr., Hansastrasse 5, D-6800 Mannheim (DE)**
Erfinder: **Fuchs, Werner, Dr., Muenchbuschweg 30B, D-6700 Ludwigshafen (DE)**

**EP 0 046 192 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur gleichzeitigen Herstellung von Pyrazol und Triazolen durch Umsetzung von Triazolinverbindungen mit basischen Verbindungen und anschliessender Oxidation mit Wasserstoffperoxid.

Es ist aus Chem. Communications, Band 1969, Seite 72, bekannt, dass man die Bis-Azoverbindung der Formel

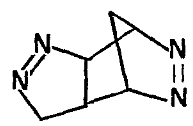

durch Erhitzen unter Stickstoff während 5 Stunden auf 120 °C in Pyrazol und einem tricyclischen Pyrazolinderivat im Mengenverhältnis 25:75 zerlegen kann:

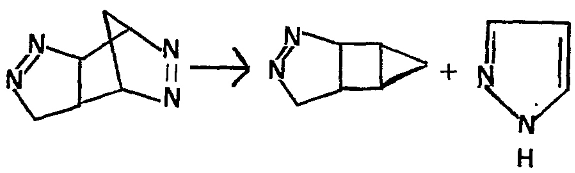

Es ist aus DE-C-557 338 bekannt, dass man Azodicarbonsäurediethylester an Cyclopentadien anlagern und das so erhaltene 3,6-Endomethylen-N,N'-dicarboxyethyltetrahydropyridazin mit Phenylazid zu 6-Phenyl-2,3-dicarbethoxy-2,3,6,7,8-pentaza-tricyclo-[5.2.1.$0^{5.9}$]-deca-7-en umsetzen kann. Weitere Umsetzungen mit diesem Decaen werden nicht beschrieben. Andere Umsetzungen von Aryl- oder Aralkylaziden mit ungesättigten zweikernigen Verbindungen werden gezeigt.

Es wird in der DE-C-557 814 gezeigt, dass zwei- oder mehrkernige Dihydrotriazole durch eine Behandlung mit Säuren in ihrem Triazolring aufgespalten werden und entsprechende Aminooxyverbindungen ergeben. Die Veröffentlichung lehrt, dass der Dihydrotriazolring des Ausgangsstoffes zunächst wahrscheinlich aufspaltet und in ein unbeständiges Diazoniumsalz übergeht. Dann soll sich unter Stickstoffabspaltung entweder durch Hydrolyse der entsprechende Aminoalkohol oder ein Ester der Aminooxyverbindung bilden. So wird z.B. die Anlagerungsverbindung von Phenylazid an N,N'-Dicarboxyethyl-3,6-endomethylentetrahydropyridazin mit verdünnter Salzsäure zu N,N'-Dicarboxyethyl-3,6-endomethylen-4-chlor-5-phenylamino-hexahydropyridazin umgesetzt.

In der DE-A-1 695 116 werden Triazolverbindungen, an deren Triazolring ein nichtaromatischer Ring anelliert ist, und ihre Herstellung, z.B. durch Reduktion entsprechender Triazoloxide, gezeigt. Die Verwendung von Triazolverbindungen als Lichtschutzmittel wird beschrieben.

Es wurde nun gefunden, dass man Pyrazol und Triazole der Formel

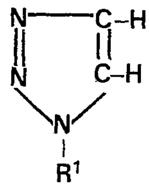

I

worin $R^1$ ein Wasserstoffatom, einen aliphatischen, cycloaliphatischen, araliphatischen oder aromatischen Rest bedeutet, gleichzeitig vorteilhaft erhält, wenn man in einem ersten Schritt Triazolinverbindungen der Formel

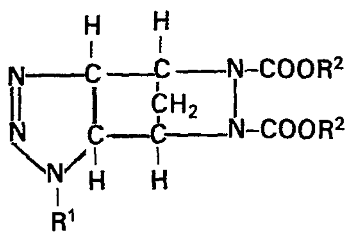

II

worin $R^1$ die vorgenannte Bedeutung besitzt und $R^2$ ein Wasserstoffatom oder einen aliphatischen Rest bezeichnet, mit basischen Verbindungen umsetzt und dann in einem 2. Schritt mit Wasserstoffperoxid, anorganischen oder organischen Peroxoverbindungen wie Natriumperoxid, Kaliumperoxid, Magnesiumperoxid, Calciumperoxid, Zinkperoxid, Bariumperoxid, Bariumsuperoxid, Hydroperoxiden wie $NaOOH \cdot 0{,}5\ H_2O_2$, $NH_4OOH$, entsprechenden Hydraten wie $CaO_2 \cdot 8H_2O$, Peroxohydraten wie $BaO_2 \cdot H_2O_2$, $BaO_2 \cdot 2H_2O_2$, Peroxocarbonaten wie Natriumperoxocarbonat, Calciumperoxocarbonat, Peroxophosphaten wie Kaliumperoxodiphosphat, und/oder Wasserstoffperoxidanlagerungsverbindungen wie Natriumboratperoxohydrat, Natriumcarbonatperoxohydrat,oxidiert und aus dem Reaktionsgemisch Pyrazol und den Endstoff I in üblicher Weise abtrennt.

Die Umsetzung kann für den Fall der Verwendung von 6-Phenyl-2,3-dicarbomethoxy-2,3,6,-7,8-pentaza-tricyclo-[5.2.1.$0^{5.9}$]-deca-7-en und Natronlauge durch die folgenden Formeln beschrieben werden:

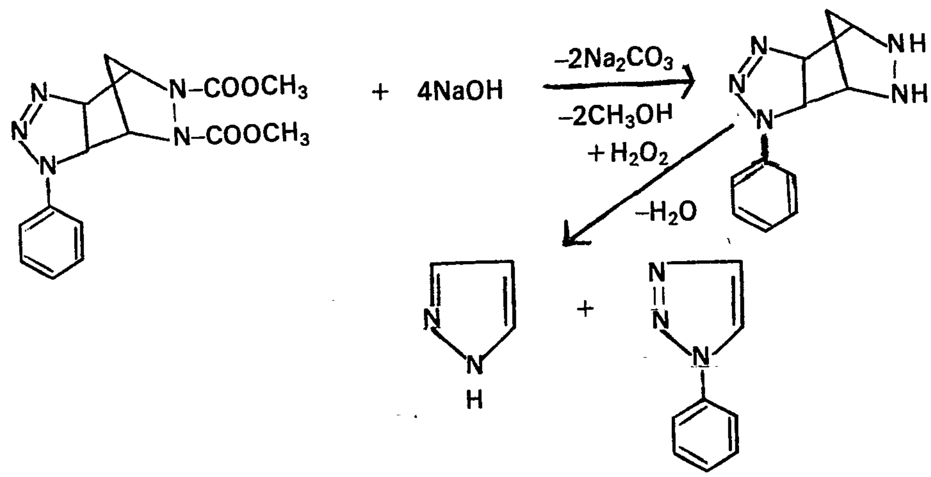


Im Vergleich zu dem bekannten Verfahren liefert das Verfahren nach der Erfindung auf einfachem und wirtschaftlichem Wege gleichzeitig Pyrazol und Triazole und das Pyrazol in besserer Ausbeute und Reinheit. Alle diese vorteilhaften Ergebnisse sind im Hinblick auf den Stand der Technik überraschend.

Die Ausgangsstoffe II sind leicht durch Addition von organischen Aziden an N,N'-Dialkoxycarbonyl-2,3-diaza-bicyclo-[2,2,1]-heptene zugänglich (DE-PS 557 338) oder im Falle von Ausgangsstoffen mit $R^1$ in der Bedeutung von Wasserstoff durch Addition von Trimethylsilylazid an vorgenannte Bicycloheptene mit anschliessender Äthanolyse des Siliciumsubstituenten.

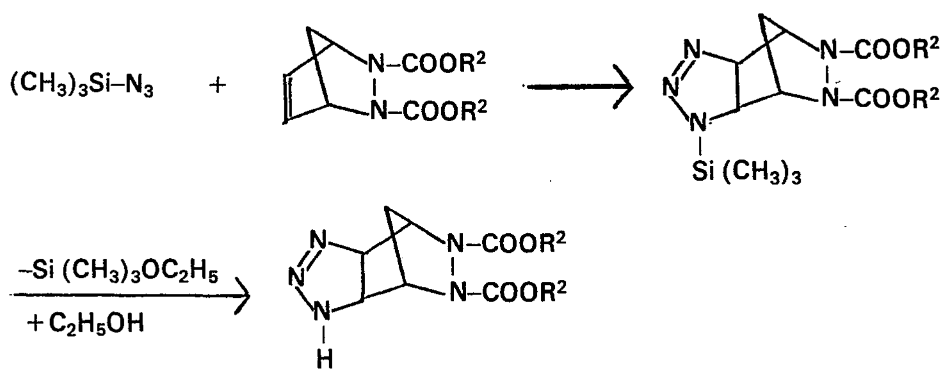


Bevorzugte Ausgangsstoffe II und dementsprechend bevorzugte Endsotffe I sind solche, in deren Formeln die einzelnen Reste $R^1$ und $R^2$ gleich oder verschieden sein können und jeweils ein Wasserstoffatom oder einen Alkylrest mit 1 bis 18, vorteilhaft 1 bis 12, insbesondere 1 bis 4 Kohlenstoffatomen bedeuten, $R^1$ auch einen Cycloalkylrest mit 5 bis 8 Kohlenstoffatomen, einen Aralkylrest oder Alkylarylrest mit 7 bis 12 Kohlenstoffatomen oder einen unsubstituierten oder durch 1 bis 5 Bromatome, Fluoratome, Chloratome, Jodatome, Nitrogruppen, Dialkylaminogruppen und/oder Alkoxygruppen mit 1 bis 4 Kohlenstoffatomen substituierten Phenylrest oder Naphthylrest bezeichnet. Die vorgenannten Reste können noch durch unter den Reaktionsbedingungen inerte Gruppen, z.B. Halogen-, Nitro-, Alkylgruppen oder Alkoxygruppen mit jeweils 1 bis 4 Kohlenstoffatomen, substituiert sein.

Es kommen z.B. als Ausgangsstoffe II in Betracht: unsubstituiertes oder in 6-Stellung durch die Methyl-, Äthyl-, Propyl-, Isopropyl-, Butyl-, sek.-Butyl-, tert.-Butyl-, Isobutyl-, Pentyl-, Hexyl-, Heptyl-, Octyl-, Nonyl-, Decyl-, Undecyl-, Dodecyl-, Cyclohexyl-, Cyclopentyl-, Cyclopropyl-, Benzyl-, Phenyl-, 2'-, 3'- oder 4'-Chlorphenyl-, o-, m-, p-Methylphenyl-, o-, m-, p-Nitrophenyl-gruppe, Naphthyl-substituiertes 2,3-Dicarbomethoxy-2,3,6,7,8-pentaza-tricyclo-$[5.2.1.0^{5,9}]$-deca-7-en; homologe Diäthyl-, Dipropyl-, Diisopropyl-, Dibutyl-, Diisobutyl-, Di- sek.-butyl-, Di-tert.-butyl-ester vorgenannter Decene.

Die Umsetzung wird zweckmässig in beiden Stufen bei einer Temperatur von −20 bis +140, vorzugsweise von 40 bis 100 °C, drucklos oder unter Druck, kontinuierlich oder kiskontinuierlich durchgeführt. Zweckmässig verwendet man unter den Reaktionsbedingungen inerte Lösungsmittel. Als Lösungsmittel kommen z.B. in Frage: Wasser; Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol; und entsprechende Gemische. Zweckmässig verwendet man das Lösungsmittel in einer Menge von 100 bis 10 000 Gewichtsprozent, vorzugsweise von 200 bis 2 000 Gewichtsprozent, bezogen auf Ausgangsstoff II. Zweckmässig wird das Lösungsmittel oder ein Anteil an

Lösungsmittel in Gestalt der Lösung des Peroxids bzw. der basischen Verbindungen verwendet.

Der erste Schritt der Umsetzung wird mit einer basischen Verbindung, vorteilhaft in einer Menge von 4 bis 20, vorzugsweise von 4 bis 8 Äquivalenten basischer Verbindung, bezogen auf ein Mol Ausgangsstoff II, durchgeführt. Bevorzugte basische Verbindungen sind Erdalkaliverbindungen, Ammoniumverbindungen und insbesondere Alkaliverbindungen sowie entsprechende Gemische. Es kommen z.B. als basische Verbindungen in Frage: Kaliumhydroxid, Natriumhydroxid, Kaliumcarbonat, Natriumcarbonat, Lithiumhydroxid, Lithiumcarbonat, Calciumhydroxid, Calciumoxid, Bariumoxid, Bariumhydroxid.

Der 2. Schritt der Umsetzung wird mit Wasserstoffperoxid durchgeführt. Das Wasserstoffperoxid wird zweckmässig in Form seiner 5- bis 60-, vorzugsweise 10- bis 55-gewichtsprozentigen, wässrigen Lösung verwendet. Gegebenenfalls kommen auch Stoffe in Betracht, die unter den Umsetzungsbedingungen Wasserstoffperoxid bilden, z.B. anorganische oder organische Peroxoverbindungen wie Natriumperoxid, Kaliumperoxid, Magnesiumperoxid, Calciumperoxid, Zinkperoxid, Bariumperoxid, Bariumsuperoxid; Hydroperoxide wie NaOOH · 0,5 $H_2O_2$, $NH_4OOH$; entsprechende Hydrate wie $CaO_2 \cdot 8H_2O$, Peroxohydrate wie $BaO_2 \cdot H_2O_2$ und $BaO_2 \cdot 2H_2O_2$; Peroxocarbonate wie Natriumperoxocarbonat und Calciumperoxocarbonat; Peroxophosphate wie das Kaliumperoxodiphosphat. Ebenfalls können Wasserstoffperoxidanlagerungsverbindungen verwendet werden wie Natriumboratperoxohydrat, Natriumcarbonatperoxohydrat.

Die Reaktion kann wie folgt durchgeführt werden: Ein Gemisch von Ausgangsstoff II, basischer Verbindung und Lösungsmittel, wird während 1 bis 6 Stunden bei der Reaktionstemperatur des ersten Schrittes gehalten. Dann gibt man Wasserstoffperoxid, zweckmässig zusammen mit Lösungsmittel bzw. in Gestalt seiner wässrigen Lösung, zu und führt den 2. Schritt der Umsetzung bei der Reaktionstemperatur des 2. Schrittes während 0,5 bis 2 Stunden durch. Aus dem Reaktionsgemisch werden Pyrazol und der Endstoff I in üblicher Weise, z.B. durch Filtration, Ansäuern und Einengen des Filtrats, Neutralisation und Extraktion, abgetrennt.

Die nach dem Verfahren der Erfindung erhältlichen Triazole I und Pyrazol sind wertvolle Ausgangsstoffe für die Herstellung von Farbstoffen, Schädlingsbekämpfungsmitteln und Pharmazeutika. Bezüglich der Verwendung wird auf vorgenannte Veröffentlichungen und Ullmanns Encyklopädie der technischen Chemie, Band 8, Seiten 498 bis 500, verwiesen.

Die im folgenden angegebenen Teile bedeuten Gewichtsteile.

Beispiel 1

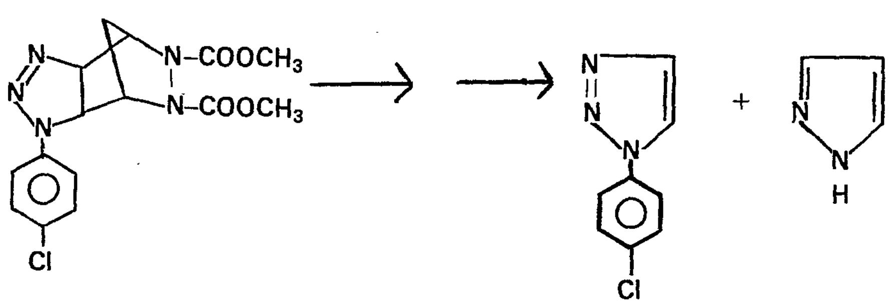

25 Teile 6-(4-Chlorphenyl)-2,3-dicarbomethoxy-2,3,6,7,8-pentaza-tricyclo-[5.2.1.$0^{5,9}$]-deca-7-en, 22 Teile 50-gewichtsprozentige, wässrige Natronlauge und 120 Teile Methanol werden unter Rühren 5 Stunden bei 68 °C erhitzt. Danach gibt man 7 Teile $H_2O_2$ in 40 Teilen Wasser bei 68 °C zu, rührt das Gemisch eine Stunde bei 68 °C nach, gibt dann 500 Teile Wasser zu und kühlt das Gemisch auf 0 °C ab. Das Filtergut wird abgesaugt, mit Wasser alkalifrei gewaschen und getrocknet. Man erhält 12 Teile (97% der Theorie) p-Chlorphenyltriazol vom Fp 111 °C. Das Filtrat wird mit 3,5-gewichtsprozentiger, wässriger Salzsäure angesäuert und im Rotationsverdampfer eingeengt (15 mbar; 40 °C). Der Rückstand wird in 100 Teilen Wasser gelöst, mit wässriger Natronlauge neutralisiert und das Gemisch viermal mit 100 Teilen $CH_2Cl_2$ extrahiert. Aus dem $CH_2Cl_2$-Extrakt erhält man durch Einengen 4,2 Teile (90% der Theorie) Pyrazol vom Fp 69 °C.

Beispiele 2 bis 12

Entsprechend Beispiel 1 werden die in der Tabelle niedergegelegten Umsetzungen durchgeführt.

Tabelle

| Beispiel | $R^1$ | Teile Ausgangsstoff II | Triazol I Ausbeute in % der Theorie | Fp (°C) oder $^1H$-NMR (δ in ppm) | Pyrazol Ausbeute in % der Theorie | Fp (°C) |
|---|---|---|---|---|---|---|
| 2 | $n-C_{12}H_{25}-$ | 50 | 93 | 44 | 90 | 69 |
| 3 | $C_6H_5-$ | 50 | 87 | 47 | 85 | 69 |
| 4 | $o-CH_3-C_6H_4$(a) | 60 | 76 | Öl, 2,2 (s, 3H) 7,3 (s, 4H), 7,7 (s, 2H) | 78 | 69 |
| 5 | $m-CH_3-C_6H_4$(a) | 50 | 71 | Öl, 2,35 (s, 3H), 7,3 (m, 4H), 7,7 (s, 1H), 7,9 (s, 1H) | 70 | 69 |
| 6 | 1-Naphthyl | 20 | 90 | 56 | 88 | 69 |
| 7 | Cyclohexyl | 20 | 95 | 34 | 91 | 69 |
| 8 | Benzyl | 15 | 79 | 60 | 70 | 69 |
| 9 | Methyl (b) | 50 | 82 | 228 | 79 | 69 |
| 10 | n-Propyl (b) | 40 | 87 | $Kp_{14}$ 113 °C | 83 | 69 |
| 11 | 2,4-Dichlorphenyl | 20 | 89 | 80 | 84 | 69 |
| 12 | 4-Nitrophenyl | 20 | 78 | 203 | 75 | 69 |

(a) Diese Verbindungen scheiden sich beim Abkühlen als Öle ab.
(b) Diese Verbindungen werden durch Extraktion mit $CH_2Cl_2$ isoliert.
Beispiel aus Chem. Communications, 1969, 72: Pyrazoline (Ausgangsstoff: Pyrazolinverbindung)

**Patentanspruch**

Verfahren zur gleichzeitigen Herstellung von Pyrazol und Triazolen der Formel

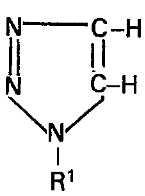

I

worin $R^1$ ein Wasserstoffatom, einen aliphatischen, cycloaliphatischen, araliphatischen oder aromatischen Rest bedeutet, dadurch gekennzeichnet, dass man in einem ersten Schritt Triazolinverbindungen der Formel

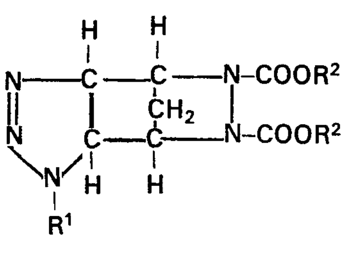

II

worin $R^1$ die vorgenannte Bedeutung besitzt und $R^2$ ein Wasserstoffatom oder einen aliphatischen Rest bezeichnet, mit basischen Verbindungen umsetzt und dann in einem 2. Schritt mit Wasserstoffperoxid, anorganischen oder organischen Peroxoverbindungen wie Natriumperoxid, Kaliumperoxid, Magnesiumperoxid, Calciumperoxid, Zinkperoxid, Bariumperoxid, Bariumsuperoxid, Hydroperoxiden wie $NaOOH \cdot 0,5\ H_2O_2$, $NH_4OOH$, entsprechenden Hydraten wie $CaO_2 \cdot 8H_2O$, Peroxohydraten wie $BaO_2 \cdot H_2O_2$, $BaO_2 \cdot 2H_2O_2$, Peroxocarbonaten wie Natriumperoxocarbonat, Calciumperoxocarbonat, Peroxophosphaten wie Kaliumperoxodiphosphat, und/oder Wasserstoffperoxidanlagerungsverbindungen wie Natriumboratperoxohydrat, Natriumcarbonatperoxohydrat, oxidiert und aus dem Reaktionsgemisch Pyrazol und den Endstoff I in üblicher Weise abtrennt.

**Revendication**

Procédé pour la préparation simultanée du pyrazole et de triazoles de formule

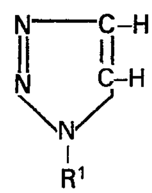

I

dans laquelle $R^1$ représente un atome d'hydrogène, un reste aliphatique, cycloaliphatique, araliphatique ou aromatique, caractérisé en ce que, dans un premier stade opératoire, on fait réagir des dérivés de la triazoline de formule

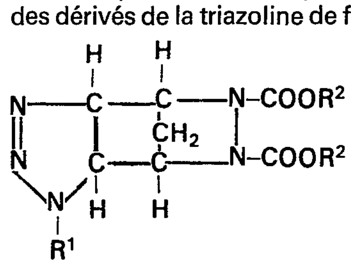

II

dans laquelle $R^1$ a la signification indiquée ci-dessus et $R^2$ représente un atome d'hydrogène ou un reste aliphatique et, dans un deuxième stade opératoire, on oxyde à l'aide du peroxyde d'hydrogène, de composés minéraux ou organiques en peroxo comme le peroxyde de sodium, le peroxyde de potassium, le peroxyde de magnésium, le peroxyde de calcium, le peroxyde de zinc, le peroxyde de baryum, le superoxyde de baryum, des hydroperoxydes tels que $NaOOH \cdot 0,5\ H_2O_2$, $NH_4OOH$, les hydrates correspondants tels que $CaO_2 \cdot 8H_2O$, des peroxohydrates tels que $BaO_2 \cdot H_2O_2$, $BaO_2 \cdot 2H_2O_2$, des peroxocarbonates comme le peroxocarbonate de sodium, le peroxocarbonate de calcium, des peroxophosphates comme le peroxodiphosphate de potassium, et/ou des composés d'addition du peroxyde d'hydrogène comme le peroxohydrate du borate de sodium, le peroxohydrate de carbonate de sodium, après quoi on sépare le pyrazole et le produit final I du mélange de réaction de la manière habituelle.

## Claim

A process for the simultaneous preparation of pyrazole and triazoles of the formula

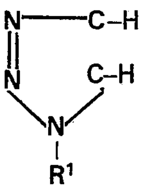

I

where $R^1$ is hydrogen or an aliphatic, cycloaliphatic, araliphatic or aromatic radical, wherein, in a first step, a triazoline compound of the formula

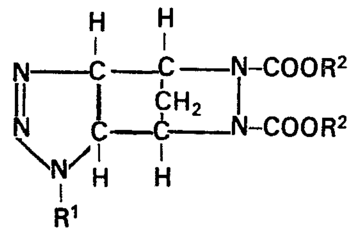

II

where $R^1$ has the above meanings and $R^2$ is hydrogen or an aliphatic radical, is reacted with a basic compound and thereafter, in a 2nd step, the product is oxidized with hydrogen peroxide; inorganic or organic peroxo compounds such as sodium peroxide, potassium peroxide, magnesium peroxide, calcium peroxide, zinc peroxide, barium peroxide; hydroperoxides, eg. $NaOOH \cdot 0.5\ H_2O_2$ and $NH_4OOH$; corresponding hydrates, such as $CaO_2 \cdot 8H_2O$ and peroxo-hydrates, such as $BaO_2 \cdot H_2O_2$ and $BaO_2 \cdot 2H_2O_2$; peroxocarbonates, such as sodium peroxocarbonate and calcium peroxocarbonate; peroxophosphates, such as potassium peroxodiphosphate, and/or hydrogen peroxide adducts, such as sodium borate peroxohydrate and sodium carbonate peroxohydrate, and pyrazole and the end product I are separated from the reaction mixture in a conventional manner.